(19) Europäisches Patentamt
European Patent Office
Office européen des brevets

(11) Veröffentlichungsnummer: **0 425 906 B1**

(12) **EUROPÄISCHE PATENTSCHRIFT**

(45) Veröffentlichungstag der Patentschrift: **02.03.94**

(51) Int. Cl.5: **C07D 209/88**, A61K 31/40

(21) Anmeldenummer: **90119974.5**

(22) Anmeldetag: **18.10.90**

Die Akte enthält technische Angaben, die nach dem Eingang der Anmeldung eingereicht wurden und die nicht in dieser Patentschrift enthalten sind.

(54) **Substituierte Cycloalkano[b]dihydroindol- und -indolsulfonamide.**

(30) Priorität: **30.10.89 GB 8924392**

(43) Veröffentlichungstag der Anmeldung: **08.05.91 Patentblatt 91/19**

(45) Bekanntmachung des Hinweises auf die Patenterteilung: **02.03.94 Patentblatt 94/09**

(84) Benannte Vertragsstaaten: **AT BE CH DE DK ES FR GB GR IT LI LU NL SE**

(56) Entgegenhaltungen:
**EP-A- 0 201 735**
**EP-A- 0 234 708**
**EP-A- 0 242 518**
**EP-A- 0 242 767**
**EP-A- 0 310 179**

(73) Patentinhaber: **BAYER AG**

**D-51368 Leverkusen(DE)**

(72) Erfinder: **Böshagen, Horst, Dr.**
**Wiesenstrasse 4**
**W-5657 Haan(DE)**
Erfinder: **Müller, Ulrich, Dr.**
**Claudiusweg 5**
**W-5600 Wuppertal 1(DE)**
Erfinder: **Rosentreter, Ulrich, Dr.**
**Kondorweg 23**
**W-5600 Wuppertal 1(DE)**
Erfinder: **Bischoff, Erwin, Dr.**
**Pahlkestrasse 73**
**W-5600 Wuppertal 1(DE)**
Erfinder: **Fiedler, Volker-Bernd, Dr.**
**Lehner Mühle 46**
**W-5090 Leverkusen 3(DE)**
Erfinder: **Perzborn, Elisabeth, Dr.**
**Am Tescher Busch 13**
**W-5600 Wuppertal 11(DE)**
Erfinder: **Hütter, Joachim, Dr.**
**Weinhauserstrasse 9**
**W-5090 Leverkusen 1(DE)**
Erfinder: **Norman, Peter, Dr.**
**4 St. Andrews Way,**
**Cippenham**
**Slough Berks, SL1 5NX(GB)**
Erfinder: **Cuthbert, Nigel J., Dr.**
**Langtree,**
**Clarendon Road,**
**Prestwood**
**Great Missenden, Bucks, HP16 0PL(GB)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

Erfinder: **Francis, Hilary P., Dr.**
**38 Eastwood Road**
**Woodley, Berks. RG5 3PY(GB)**
Erfinder: **McKenniff, Marie G., Dr.**
**21 Charles Gardens**
**Slough, Berks, SL2 5QX(GB)**

## Beschreibung

Die Erfindung betrifft substituierte Cycloalkano[b]-indolsulfonamide, Verfahren zu ihrer Herstellung und ihre Verwendung in Arzneimitteln.

Es ist bereits bekannt geworden, daß Cycloalkano[b]-indolsulfonamide eine thrombozytenaggregationshemmende Wirkung aufweisen (vergl. DOS 36 31 824).

Es wurden nun substituierte Cycloalkano[b]-indolsulfonamide der allgemeinen Formel (I)

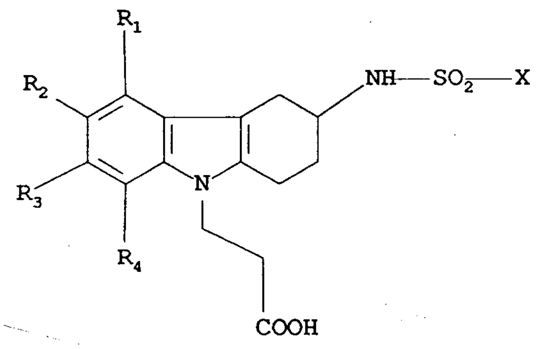

worin

| | |
|---|---|
| $R_1$, $R_2$, $R_3$ und $R_4$ | gleich oder verschieden sind und für Wasserstoff, Fluor, Chlor, Brom oder Jod stehen, und |
| X | für Phenyl steht, das gebenenfalls bis zu 4-fach gleich oder verschieden durch Nitro, Fluor, Chlor, Brom, Jod, Cyano, Trifluormethyl, Trifluormethoxy, Hydroxy, Carboxy, Phenyl, Phenoxy, geradkettiges oder verzweigtes Alkoxy oder Alkoxycarbonyl mit jeweils bis zu 6 Kohlenstoffatomen oder durch geradkettiges oder verzweigtes Alkyl mit bis zu 6 Kohlenstoffatomen substituiert sein kann, oder für geradkettiges oder verzweigtes Alkyl mit bis zu 6 Kohlenstoffatomen oder für Trifluormethyl steht, |

mit der Maßgabe, daß stets zwei der Substituenten $R_1$ - $R_4$ verschieden von Wasserstoff sind, gegebenenfalls in einer isomeren Form und ihre Salze, gefunden.

Die erfindungsgemäßen substituierten Cycloalkano[b]-indolsulfonamide haben eines oder mehrere asymmetrische Kohlenstoffatome und können daher in verschiedenen stereochemischen Formen existieren. Außerdem können Regioisomere auftreten. Sowohl die einzelnen Isomeren als auch deren Mischungen sind Gegenstand der Erfindung.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vergl. E. L. Eliel, Stereochemistry of Carbon Compounds, McGraw Hill, 1962).

Im folgenden werden beispielhaft isomere Formen der Cycloalkano[b]-indolsulfonamide aufgeführt:

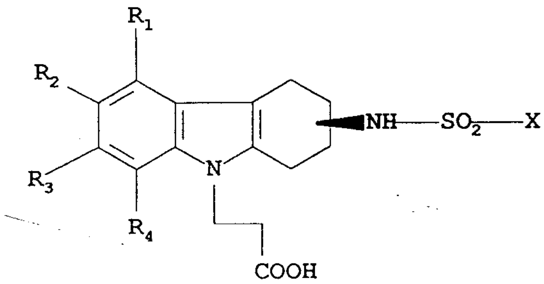

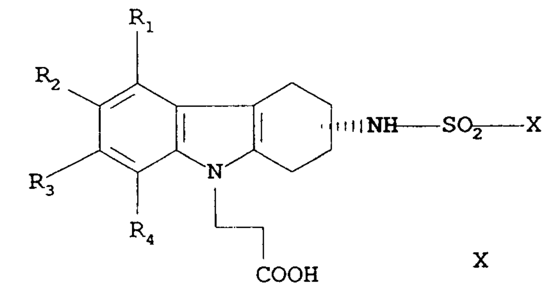

X

Die erfindungsgemäßen substituierten Cycloalkano[b]-indolsulfonamide können auch in Form ihrer Salze vorliegen. Im allgemeinen seien hier Salze mit organischen oder anorganischen Basen genannt.

Im Rahmen der vorliegenden Erfindung werden physiologisch unbedenkliche Salze bevorzugt. Physiologisch unbedenkliche Salze der substituierten Cycloalkano[b]-indolsulfonamide können Metall- oder Ammoniumsalze der erfindungsgemäßen Stoffe, welche eine freie Carboxylgruppe besitzen, sein. Besonders bevorzugt sind z. B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Stoffe zeigen überraschenderweise eine thrombozytenaggregationshemmende Wirkung, bewirken außerdem eine Hemmung der Thromboxansynthese an isolierten Plättchen.

Bevorzugt sind Verbindungen der allgemeinen Formel (I), in welcher

$R_1$, $R_2$, $R_3$ und $R_4$ gleich oder verschieden sind und für Wasserstoff, Fluor oder Chlor stehen, und

X für Phenyl steht, das gegebenenfalls bis zu 3-fach gleich oder verschieden durch Fluor, Chlor, Brom, Cyano, Trifluormethyl, Trifluormethoxy oder durch geradkettiges oder verzweigtes Alkyl, Alkoxy oder Alkoxycarbonyl mit jeweils bis zu 4 Kohlenstoffatomen substituiert ist,

mit der Maßgabe, daß stets zwei der Substituenten $R_1$ bis $R_4$ verschieden sind von Wasserstoff, gegebenenfalls in einer isomeren Form und ihre Salze.

Die Verbindungen der allgemeinen Formel (I) können hergestellt werden, indem man

[A] Verbindungen der allgemeinen Formel

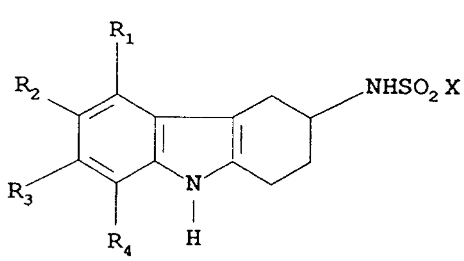

VIII

in welcher

$R_1$, $R_2$, $R_3$ , $R_4$ und X die angegebene Bedeutung haben mit Acrylnitril, gegebenenfalls in Anwesenheit

einer Base in inerten Lösemitteln zunächst zu den entsprechenden Cyanoethylverbindungen umsetzt und anschließend zu den entsprechenden Säuren verseift,
oder indem man
[B] Verbindungen der allgemeinen Formel

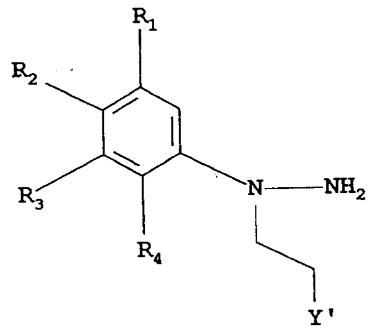

in welcher
$R_1$, $R_2$, $R_3$ und $R_4$ die angegebene Bedeutung haben, und
Y' für ($C_1$-$C_4$)-Alkoxycarbonyl oder Cyano steht,
mit Cycloalkanosulfonamiden der allgemeinen Formel

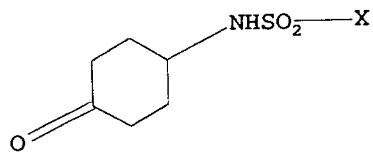

in welcher
X die angegebene Bedeutung hat
in inerten Lösemitteln, gegebenenfalls in Anwesenheit eines Katalysators umsetzt, und die Ester nach üblicher Methode verseift, gegebenenfalls die Isomeren trennt und im Fall der Herstellung der Salze mit einer entsprechenden Base umsetzt.

Das erfindungsgemäße Verfahren kann durch folgendes Formelschema beispielhaft erläutert werden:

[A]

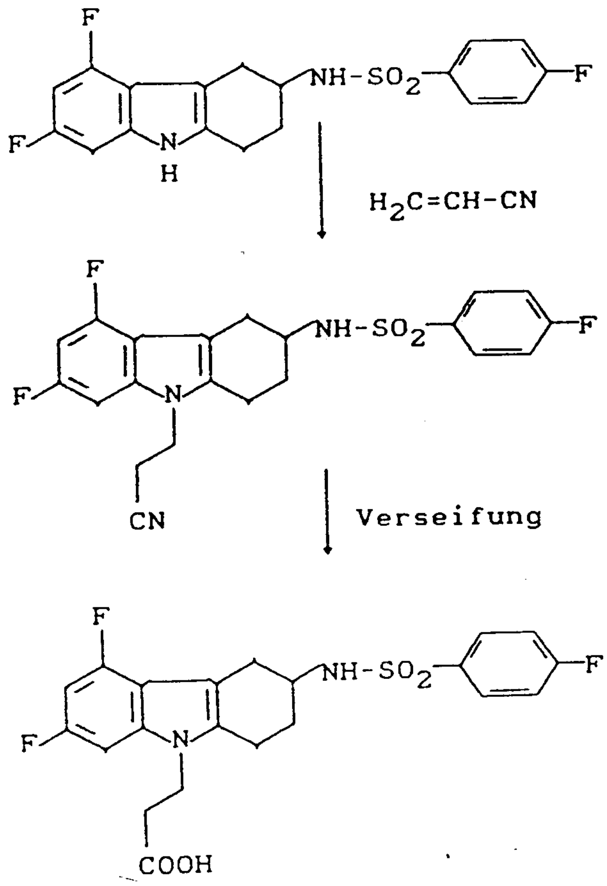


[B] Beispielhaft, nicht erfindungsgemäß

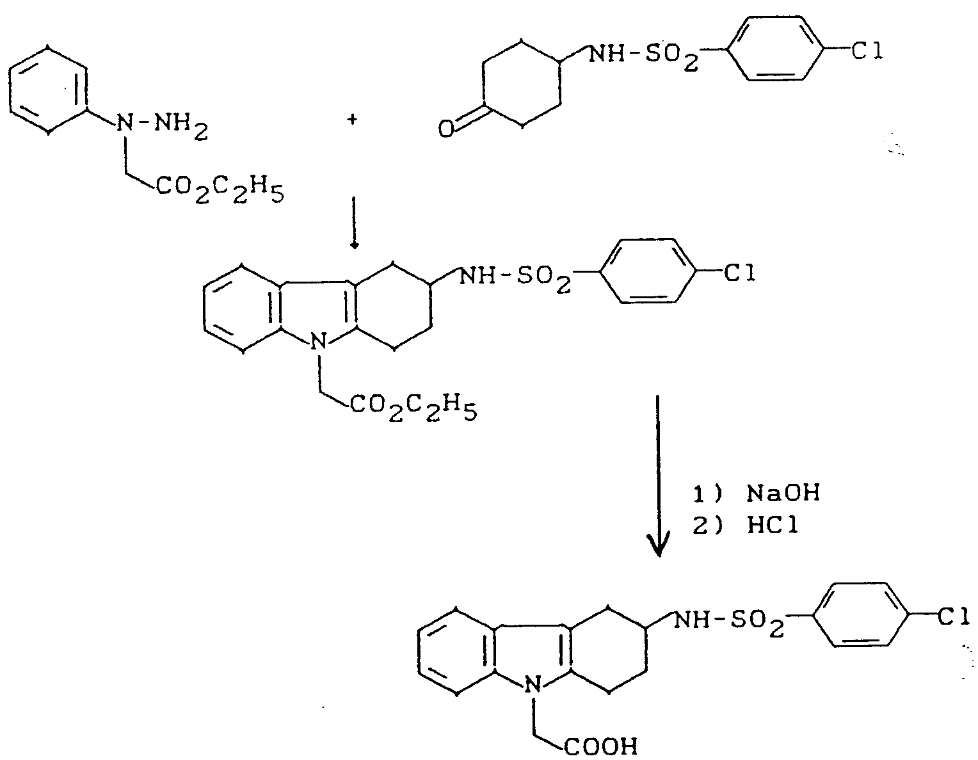

Lösungsmittel für die erfindungsgemäßen Verfahren [A] und [B] können Wasser und organische Lösungsmittel sein, die sich unter den Reaktionsbedingungen nicht verändern. Hierzu gehören bevorzugt chlorierte Kohlenwasserstoffe, wie beispielsweise Chloroform oder Methylenchlorid, Alkohole wie Methanol, Ethanol, Propanol oder Isopropanol, Ether wie Diethylether, Tetrahydrofuran, Dioxan, Glykolmono- oder -dimethylether, Kohlenwasserstoffe wie Benzol, Toluol, Xylol, Cyclohexan, Hexan oder Erdölfraktionen, Dimethylsulfoxid, Dimethylformamid, Hexamethylphosphorsäuretriamid, Essigester, Acetonitril oder Pyridin. Ebenso ist es möglich, Gemische der genannten Lösungsmittel zu verwenden.

Basen für die erfindungsgemäßen Verfahren [A] und [B] können übliche basische Verbindungen sein. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat-, -ethanolat oder Kalium-tert.-butylat oder Amide wie Natriumamid oder Lithiumdiisopropylamid, oder organische Amine oder Ammoniumsalze wie Benzyltrimethylammoniumhydroxid, Tetrabutylammoniumhydroxid, Pyridin, Triethylamin oder N-Methylpiperidin.

Die erfindungsgemäßen Verfahren [A] und [B] werden im allgemeinen in einem Temperaturbereich von 0 °C bis 150 °C, bevorzugt von 0 °C bis 100 °C, durchgeführt.

Im allgemeinen werden die Verfahren [A] und [B] bei Normaldruck durchgeführt. Es ist aber auch möglich, bei Unterdruck oder bei Überdruck zu arbeiten (z.B. von 0,5 bis 5 bar).

Die Verseifung der Ester erfolgt nach üblicher Methode, indem man die Ester in inerten Lösemitteln mit üblichen Basen behandelt, wobei die zunächst entstehenden Salze durch Behandeln mit Säure in die freien Carbonsäuren überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0 °C bis +140 °C, bevorzugt von +20 °C bis +100 °C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 5 mol, bevorzugt von 1 bis 2 mol bezogen auf 1 mol des Esters eingesetzt. Besonders bevorzugt verwendet man molare Mengen der Reaktanden.

Bei der Durchführung der Reaktion entstehen im ersten Schritt die Salze der erfindungsgemäßen Verbindungen als Zwischenprodukte, die isoliert werden können. Die erfindungsgemäßen Säuren erhält man durch Behandeln der Salze mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren hierbei als vorteilhaft erwiesen, die basische Reaktionsmischung der Verseifung in einem zweiten Schritt ohne Isolierung der Salze anzusäuern. Die Säuren können dann in üblicher Weise isoliert werden.

Die Verbindungen der allgemeinen Formel (VIII) sind teilweise neu. Sie können hergestellt werden, indem man

Phenylhydrazine der allgemeinen Formel (XII)

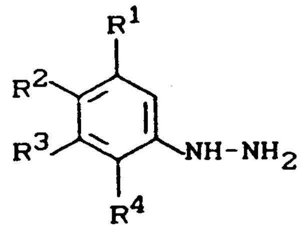

(XII)

in welcher

$R^1$, $R^2$, $R^3$ und $R^4$ die oben angegebene Bedeutung haben,

mit Cycloalkanonsulfonamiden der allgemeinen Formel (X)

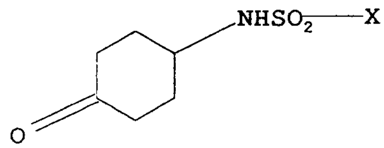

in welcher

X die oben angegebene Bedeutung hat,

in Gegenwart von den oben aufgeführten inerten Lösemitteln, gegebenenfalls in Anwesenheit eines Katalysators, umsetzt.

Die Umsetzung mit Phenylhydrazinen der Formel (XII) verläuft unter den bei Verfahren [B] beschriebenen Reaktionsbedingungen.

Als Hydrazine für das erfindungsgemäße Verfahren werden beispielsweise eingesetzt: Phenylhydrazin, 4-Methoxyphenylhydrazin, 4-Chlorphenylhydrazin, 4-Fluorphenylhydrazin, 4-Methylphenylhydrazin, 2,4-Difluorphenylhydrazin, 3,5-Difluorphenylhydrazin, 3-Fluorphenylhydrazin und 2-Fluorphenylhydrazin.

Die Phenylhydrazine der allgemeinen Formel (XII) sind teilweise bekannt oder können nach üblicher Methode hergestellt werden [vgl. Houben-Weyl, "Methoden der organischen Chemie" X/2, Seite 1, 123, 693].

Die Cycloalkanonsulfonamide der allgemeinen Formel (X) und ihre Darstellung sind bekannt [vgl. DOS 36 31 824].

Die erfindungsgemäßen enantiomerenreinen Verbindungen der allgemeinen Formel (I) können nach üblichen Methoden, z.B. in Analogie zu dem in der DOS 36 31 824 beschriebenen Verfahren erhalten werden.

Die Verbindungen der allgemeinen Formel (IX) sind an sich bekannt oder können nach üblicher Methode hergestellt werden [vgl. DOS 23 12 256].

Die substituierten Cycloalkano[b]indolsulfonamide bzw. deren Salze können als Wirkstoffe in Arzneimitteln eingesetzt werden. Die Wirkstoffe weisen eine thrombozytenaggregationshemmende und Thromboxan $A_2$-antagonistische Wirkung auf und bewirken eine Hemmung der Thromboxansynthase an isolierten Plättchen. Sie können bevorzugt zur Behandlung von Thrombosen, Thromboembolien, Ischämien, sowie zur Prophylaxe des Myocardinfarktes als Antiasthmatika und als Antiallergika eingesetzt werden.

Zur Bestimmung der thrombozytenaggregationshemmenden Wirkung wurde Blut von gesunden Probanden beiderlei Geschlechts verwendet. Als Antikoagulans wurden einem Teil 3,8%iger wäßriger Natriumzitratlösung 9 Teile Blut zugemischt. Mittels Zentrifugation erhält man aus diesem Blut plättchenreiches Zitratplasma (PRP)[1] (Jürgens/ Beller, Klinische Methoden der Blutgerinnungsanalyse; Thieme Verlag, Stuttgart, 1959).

Für diese Untersuchungen wurden 0,8 ml (PRP)[1] und 0,1 ml der Wirkstofflösung bei 37°C im Wasserbad vorinkubiert. Anschließend wurde die Thrombozytenaggregation nach der turbidometrischen Methode (Born, G.V.R., J. Physiol. (London), 162, 67, 1962) im Aggregometer bei 37°C bestimmt (Therapeutische Berichte 47, 80-86, 1975). Hierzu wurde die vorinkubierte Probe mit 0,1 ml Kollagen, einem aggregationsauslösenden Agens, versetzt. Die Veränderung der optischen Dichte in der Probe der (PRP)[1] wurde während einer Zeitdauer von 6 Minuten aufgezeichnet und der Ausschlag nach 6 Minuten bestimmt. Hierzu wird die prozentuale Hemmung gegenüber der Kontrolle errechnet. Als Grenzkonzentration wird der Bereich der minimal effektiven Konzentration angegeben.

Die Grenzkonzentrationen liegen zwischen 0,01 - 10 mg/l.

| Beispiel-Nr. | EC mg/l |
|---|---|
| 4 | 0,01 - 0,03 |

Messung der Thromboxansynthase an gewaschenen Humanthrombozyten

1. Präparation von Thrombozytensuspensionen

Blut von gesunden Spendern wird in EDTA (1% in 0,9% NaCl, 9 + 1) aufgenommen und bei 1000 U/min (150 g) 20 min zentrifugiert. Das plättchenreiche Plasma (PRP)[2] wird abgehebert und jeweils 10 ml bei

2500 U/min 20 min zentrifugiert. Das plättchenreiche Plasma[2] wird abdekantiert. Die verbleibenden Plättchen werden in 5 ml Resuspensionspuffer (0,15 M TRIS / 0,9% NaCl/ 77 mmol EDTA, 8:91:1; mit 1 N HCl auf pH 7,4 eingestellt) suspendiert, 20 min bei 2500 U/min zentrifugiert und in 1 ml Resuspensionspuffer suspendiert. Die Thrombozytenzahl wird auf 3 x $10^5/\mu l$ eingestellt.

2. Messung der Thromboxansynthase

1 ml der Plättchensuspension und 0,01 ml des Prüfpräparates in 10% DMSO-, werden 2 min bei 37°C inkubiert. Dazu werden 0,1 ml $^3H$-Arachidonsäure Amersham Buchler GmbH und Co. KG (6,6 x $10^{-5}$ mol/l) mit einer spezifischen Aktivität von 140 MBq/mmol zugegeben und weitere 10 min bei 37°C inkubiert. Nach der Reaktion wird das Gemisch mit ca. 0,02 ml 0,5 N Citronensäure angesäuert und unmittelbar mit 3-mal mit je 1 ml Essigester extrahiert Die Überstände werden in 10 ml Glasröhrchen gesammelt und der Essigester unter $N_2$ bei 25°C abgeblasen. Der Rückstand wird in 50 $\mu l$ MeOH/$CHCl_3$ (1:1) aufgenommen und auf DC-Glasplatten (Kieselgel 60 F254 20 x 20 cm, Merck) aufgetragen.

Die Trennung erfolgt in einem Fließmittelgemisch $CHCl_3$/MeOH/Eisessig/$H_2O$ (80:8:1:0,8). Die Verteilung der Radioaktivität wird in einem DC-Scanner Ramona-Ls der Fa. Raytest erfaßt und mit einem Integrationsprogramm quantitativ ausgewertet.

Bestimmt wird die Konzentration der Prüfsubstanzen, die zu einer 50% Hemmung der Thromboxanbildung gegenüber der Kontrolle führt.

Hemmung der Thromboxansynthese an gewaschenen Plättchen aus Humanblut.

| Beispiel-Nr. | $IC_{50}$ mol/l |
|---|---|
| 4 | 1-3 x $10^{-6}$ |

Die neuen Wirkstoffe können in an sich bekannter Weise unter Verwendung inerter nichttoxischer, pharmazeutisch geeigneter Trägerstoffe oder Lösungsmittel in die üblichen Formulierungen überführt werden, wie Tabletten, Kapseln, Dragees, Pillen, Granulate, Aerosole, Sirupe, Emulsionen, Suspensionen und Lösungen. Hierbei soll die therapeutisch wirksame Verbindung jeweils in einer Konzentration von etwa 0,5 bis 90 Gew.-%, bevorzugt von 5 bis 70 Gew.-%, bezogen auf die Zubereitung, vorliegen, die ausreichend ist, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nichttoxische organische Lösungsmittel wie Paraffine (z. B. Erdölfraktionen), pflanzliche Öle (z. B. Erdnuß/Sesamöl), Alkohole (z.B. Ethylalkohol, Glycerin), Glykole (z.B. Propylenglykol, Propylethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäureester, Polyoxyethylen-Fettalkohol-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation kann in üblicher Weise erfolgen, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den oben genannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen. Bei oraler Applikation beträgt die Dosierung im allgemeinen etwa 0,01 bis 20 mg/kg, vorzugsweise 0,1 bis 10 mg/kg Körpergewicht.

Es kann aber gegebenenfalls vorteilhaft sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehrere Einzelgaben über den Tag zu verteilen.

Ausgangsverbindungen

Beispiel I

3,5-Difluorphenylhydrazin

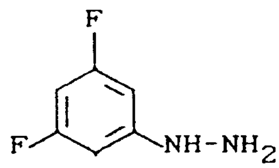

30,50 g (2,363 mmol) 3,5-Difluoranilin werden in 128 ml (700 mmol) 20%iger HCl suspendiert und bei Temperaturen unter 5°C mit 16,46 g (236,3 mmol) Natriumnitrit in 95 ml Wasser verrührt. Zur fast vollständigen Auflösung des Feststoffes wird 30 Minuten bei 0°C nachgerührt. Die kalte Lösung wird portionsweise unter Kühlung zu 769 g (2,95 mol) einer 40%igen Natriumbisulfitlösung gegeben, wobei mit 2 N Natronlauge auf einem pH-Wert von 6,5 gehalten wird. Hierauf kocht man 4 h unter Rückfluß, wobei der pH-Wert von 6,5 alle 30 Minuten überprüft und gegebenenfalls neu eingestellt wird. Nach dem Abkühlen wird mit 1 N Natronlauge alkalisch gestellt und 5 mal mit je 400 ml $CH_2Cl_2$ extrahiert, mit $Na_2SO_4$ getrocknet, das Lösemittel abgedampft, der Rückstand mit Petrolether verrührt und im Hochvakuum getrocknet.
Ausbeute: 17,34 g (120,3 mmol; 51% der Theorie)
Fp.°C: 93-95°C
$R_f$ = 0,9 (Essigester)

Beispiel II

3-(4-Chlorphenylsulfonamido)-5,7-difluor-1,2,3,4-tetrahydrocarbazol

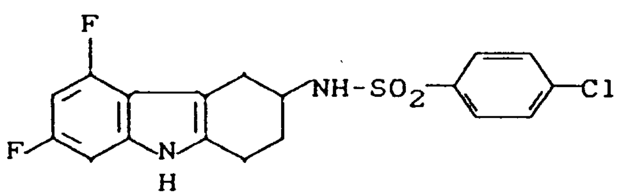

14,0 g (97 mmol) der Verbindung aus Beipiel I und 27,95 g (97 mmol) 4-Chlor-N-(4-oxo-cyclohexyl)-benzolsulfonsäureamid werden in 190 ml Ethanol und 36 ml konzentrierter Schwefelsäure 5 Stunden unter Rückfluß gekocht, abgekühlt und mit 300 ml Wasser verdünnt. Man extrahiert 4 mal mit je 250 ml Essigester, trocknet die organische Phase mit Natriumsulfat, saugt über Kieselgur ab und dampft ein. Der Rückstand wird mit Methylenchlorid verrührt und im Hochvakuum getrocknet.
Ausbeute: 37,6 g (95 mmol; 98% der Theorie)
Fp.: 171-174°C

Beispiel III

3-(4-Chlorphenylsulfonamido)-9-(2-cyanoethyl)-5,7-difluor-1,2,3,4-tetrahydrocarbazol

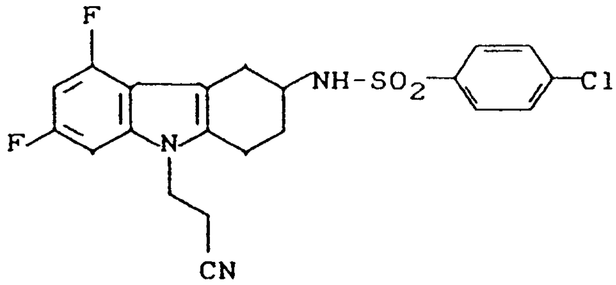

Unter Argon werden 5,95 g (15,0 mmol) der Verbindung aus Beispiel II in 30 ml Dimethylformamid p.a. gelöst und unter Rühren bei Raumtemperatur mit 495 mg (16,5 mmol) 80%igem Natriumhydrid (mit Paraffinöl) versetzt. Nach beendeter Wasserstoffentwicklung werden 1,75 g (33,0 mmol) Acrylnitril zugesetzt und 30 Minuten gerührt. Ist die Ausgangsverbindung nicht völlig verschwunden (DC-Kontrolle / Toluol:Essigester = 4:1) werden weitere 0,23 g (4,3 mmol) Acrylnitril zugegeben und noch einmal 30 Minuten gerührt. Man versetzt mit 50 ml Essigester und schüttelt aus 3 mal mit je 50 ml 1N Schwefelsäure, trocknet die organische Phase mit Natriumsulfat und dampft ab. Der Rückstand wird an Kieselgel 60 (Merck / Korngröße 40-63 µm / Laufmittel zuerst Toluol, dann steigende Essigesteranteile bis zu reinem Essigester) chromatographiert.
Ausbeute: 4,50 g (10,0 mmol; 67% der Theorie)
Fp. °C: 114-117 °C
$R_f$ = 0,24 (Toluol/Essigester = 4:1)

Herstellungsbeispiele (Formel I)

Beispiel 1

3-(4-Chlorphenylsulfonamido)-9-(2-carboxyethyl)-5,7-difluor-1,2,3,4-tetrahydrocarbazol

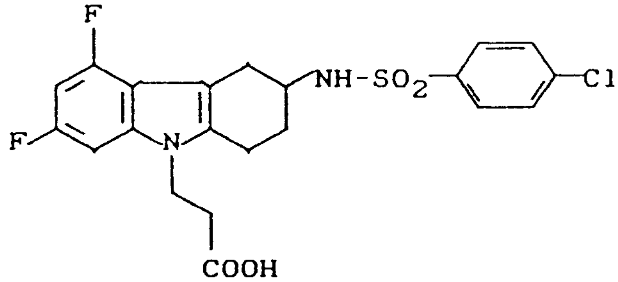

3,05 g (6,8 mmol) der Verbindung aus Beispiel III werden in 20 ml Ethanol gelöst und nach Zugabe von 100 ml 10%iger Natronlauge 16 h am Rückfluß gerührt. Der abgekühlte Ansatz wird 2 mal mit je 50 ml Methylenchlorid ausgeschüttelt und bei 0 °C mit 6 N Salzsäure auf pH 1 gestellt. Der ausgefallene Niederschlag wird abgesaugt und mit Wasser neutral gewaschen, im Hochvakuum über Phosphorpentoxid und Natriumhydroxid getrocknet und ausgewogen.
Ausbeute: 2,73 g (5,8 mmol; 86% der Theorie)
Fp.: 134 °C
Analog der Vorschrift für Beispiel 1 können die in Tabelle 1 aufgeführten Verbindungen hergestellt werden:

Tabelle 1

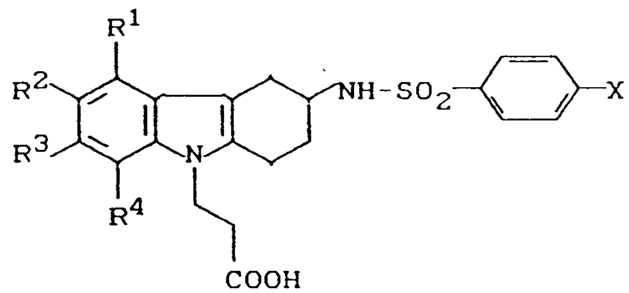

| Bsp.-Nr. | $R^1$ | $R^2$ | $R^3$ | $R^4$ | X | $R_f$-Wert* |
|---|---|---|---|---|---|---|
| 2 | F | H | F | H | F | 0,33 |
| 3 | H | F | H | F | F | 0,39 |
| 4 | H | F | H | F | Cl | 0,31 |

* Laufmittel: Dichlormethan/Methanol = 10:1

## Patentansprüche

## Patentansprüche für folgende Vertragsstaaten : AT, BE, CH, DE, DK, FR, GR, GB, IT, LU, LI, NL, SE

**1.** Substituierte Cycloalkano[b]-indolsulfonamide der Formel (I)

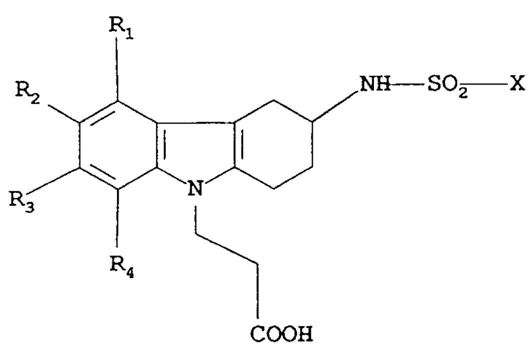

worin

$R_1$, $R_2$, $R_3$ und $R_4$ gleich oder verschieden sind und für Wasserstoff, Fluor, Chlor, Brom oder Jod stehen, und

X für Phenyl steht, das gebenenfalls bis zu 4-fach gleich oder verschieden durch Nitro, Fluor, Chlor, Brom, Jod, Cyano, Trifluormethyl, Trifluormethoxy, Hydroxy, Carboxy, Phenyl, Phenoxy, geradkettiges oder verzweigtes Alkoxy

oder Alkoxycarbonyl mit jeweils bis zu 6 Kohlenstoffatomen oder durch geradkettiges oder verzweigtes Alkyl mit bis zu 6 Kohlenstoffatomen substituiert sein kann, oder
für geradkettiges oder verzweigtes Alkyl mit bis zu 6 Kohlenstoffatomen oder für Trifluormethyl steht,

mit der Maßgabe, daß stets zwei der Substituenten $R_1$ - $R_4$ verschieden von Wasserstoff sind, gegebenenfalls in einer isomeren Form und ihre Salze.

**2.** Substitutierte Cycloalkano[b]-indolsulfonamide der Formel nach Anspruch 1 worin

$R_1$, $R_2$, $R_3$ und $R_4$ gleich oder verschieden sind und für Wasserstoff, Fluor oder Chlor stehen, und

X für Phenyl steht, das gegebenenfalls bis zu 3-fach gleich oder verschieden durch Fluor, Chlor, Brom, Cyano, Trifluormethyl, Trifluomethoxy oder durch geradkettiges oder verzweigtes Alkyl, Alkoxy oder Alkoxycarbonyl mit jeweils bis zu 4 Kohlenstoffatomen substituiert ist,

mit der Maßgabe, daß stets zwei der Substituenten $R_1$ bis $R_4$ verschieden sind von Wasserstoff, gegebenenfalls in einer isomeren Form und ihre Salze.

**3.** Substituierte Cycloalkano[b]-indolsulfonamide nach Anspruch 1 und 2 zur Bekämpfung von Krankheiten.

**4.** Verfahren zur Herstellung von substituierten Cycloalkano[b]-indolsulfonamiden nach den Ansprüchen 1 und 2, dadurch gekennzeichnet, daß man

[A] Verbindungen der allgemeinen Formel

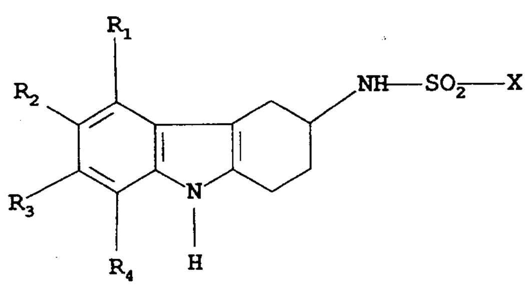

in welcher

$R_1$, $R_2$, $R_3$ , $R_4$ und X die angegebene Bedeutung haben mit Acrylnitril, gegebenenfalls in Anwesenheit einer Base in inerten Lösemitteln zunächst zu den entsprechenden Cyanoethylverbindungen umsetzt und anschließend zu den entsprechenden Säuren verseift,

oder indem man

[B] Verbindungen der allgemeinen Formel

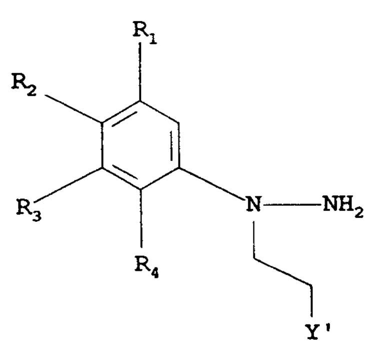

in welcher

$R_1$, $R_2$, $R_3$ und $R_4$ die angegebene Bedeutung haben, und
Y' für ($C_1$-$C_4$)-Alkoxycarbonyl oder Cyano steht,
mit Cycloalkanosulfonamiden der allgemeinen Formel

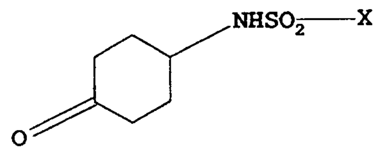

in welcher
X die angegebene Bedeutung hat
in inerten Lösemitteln, gegebenenfalls in Anwesenheit eines Katalysators umsetzt, und die Ester nach üblicher Methode verseift, gegebenenfalls die Isomeren trennt und im Fall der Herstellung der Salze mit einer entsprechenden Base umsetzt.

**5.** Arzneimittel enthaltend mindestens ein substituiertes Cycloalkano[b]-indolsufonamid nach Anspruch 1 und 2.

**6.** Arzneimittel nach Anspruch 5 zur Behandlung von Thrombosen, Thromboembolien, Ischämien, Asthma und Allergien sowie zur Prophylaxe des Myocardinfarkts.

**7.** Arzneimittel nach Anspruch 5 enthaltend 0,5 bis 90 Gew.-% der substituierten Cycloalkano[b]-indolsulfonamide nach Anspruch 1.

**8.** Verfahren zur Herstellung eines Arzneimittels nach Anspruch 6, dadurch gekennzeichnet, daß man Verbindungen nach Anspruch 1 gegebenenfalls mit Hilfe von üblichen Hilfs- und Trägerstoffen in eine geeignete Applikationsform bringt.

**9.** Verwendung der substituierten Cycloalkano[b]-indolsulfon-amide nach Anspruch 1 und 2 zur Herstellung von Arzneimitteln.

**10.** Verwendung der substituierten Cycloalkano[b]-indolsulfon-amide nach Anspruch 1 zur Herstellung von Arzneimitteln zur Behandlung von Thrombosen, Thromboembolien, Ischämien, Asthma und Allergien, sowie zur Prophylaxe des Myocardinfarktes.

**Patentansprüche für folgenden Vertragsstaat : ES**

**1.** Verfahren zur Herstellung von substituierten Cycloalkano[b]-indolsulfonamiden der Formel

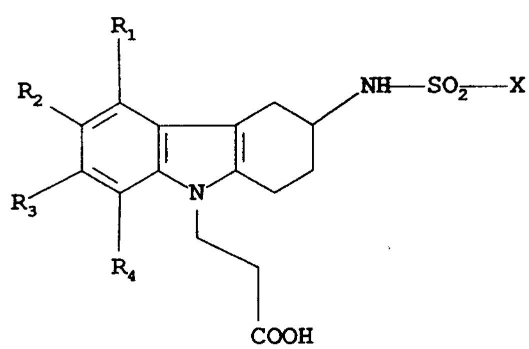

worin

| | |
|---|---|
| $R_1$, $R_2$, $R_3$ und $R_4$ | gleich oder verschieden sind und für Wasserstoff, Fluor, Chlor, Brom oder Jod stehen, und |
| X | für Phenyl steht, das gebenenfalls bis zu 4-fach gleich oder verschieden |

durch Nitro, Fluor, Chlor, Brom, Jod, Cyano, Trifluormethyl, Trifluormethoxy, Hydroxy, Carboxy, Phenyl, Phenoxy, geradkettiges oder verzweigtes Alkoxy oder Alkoxycarbonyl mit jeweils bis zu 6 Kohlenstoffatomen oder durch geradkettiges oder verzweigtes Alkyl mit bis zu 6 Kohlenstoffatomen substituiert sein kann, oder
für geradkettiges oder verzweigtes Alkyl mit bis zu 6 Kohlenstoffatomen oder für Trifluormethyl steht,

mit der Maßgabe, daß stets zwei der Substituenten $R_1$ - $R_4$ verschieden von Wasserstoff sind, gegebenenfalls in einer isomeren Form und ihre Salzen,
dadurch gekennzeichnet, daß man
[A] Verbindungen der allgemeinen Formel

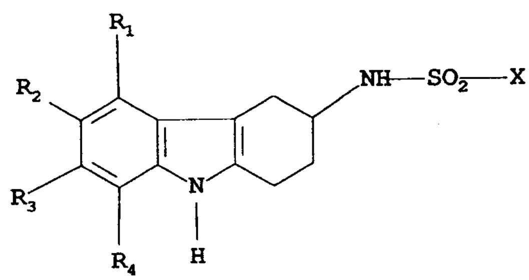

in welcher
$R_1$, $R_2$, $R_3$ , $R_4$ und X die angegebene Bedeutung haben mit Acrylnitril, gegebenenfalls in Anwesenheit einer Base in inerten Lösemitteln zunächst zu den entsprechenden Cyanoethylverbindungen umsetzt und anschließend zu den entsprechenden Säuren verseift,
oder indem man
[B] Verbindungen der allgemeinen Formel

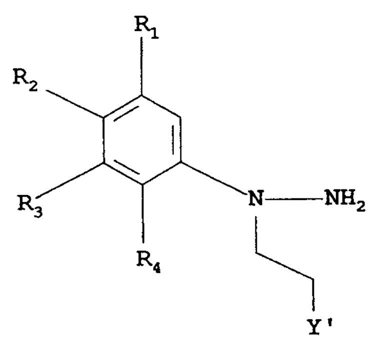

in welcher
$R_1$, $R_2$, $R_3$ und $R_4$ die angegebene Bedeutung haben, und
Y' für $(C_1-C_4)$-Alkoxycarbonyl oder Cyano steht,
mit Cycloalkanosulfonamiden der allgemeinen Formel

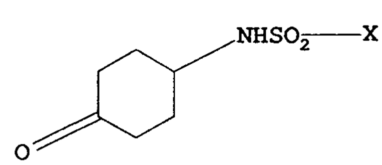

in welcher
X die angegebene Bedeutung hat
in inerten Lösemitteln, gegebenenfalls in Anwesenheit eines Katalysators umsetzt, und die Ester

nach üblicher, Methode verseift, gegebenenfalls die Isomeren trennt und im Fall der Herstellung der Salze mit einer entsprechenden Base umsetzt.

2. Verfahren nach Anspruch 1, dadurch gekennzeichnet, daß die Umsetzung mit Acrylnitril in einem Temperaturbereich von 0 - 150 °C durchgeführt wird.

3. Verfahren nach Anspruch 1, dadurch gekennzeichnet, daß die Veresterung und Amidierung über die aktive Stufe der Säurehalogenide durchgeführt wird.

4. Verfahren nach Anspruch 1, dadurch gekennzeichnet, daß die Verfahren bei Normaldruck durchgeführt werden.

**Claims**

**Claims for the following Contracting States : AT, BE, CH, DE, DK, FR, GR, GB, IT, LU, LI, NL, SE**

1. Substituted cycloalkano[b]-indolesulphonamides of the formula (I)

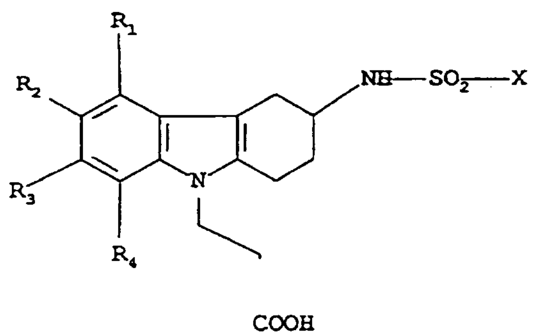

in which

| | |
|---|---|
| $R_1$, $R_2$, $R_3$ and $R_4$ | are identical or different and represent hydrogen, fluorine, chlorine, bromine or iodine, and |
| X | represents phenyl which can optionally be up to tetrasubstituted by identical or different substituents from the series comprising nitro, fluorine, chlorine, bromine, iodine, cyano, trifluoromethyl, trifluoromethoxy, hydroxyl, carboxyl, phenyl, phenoxy, straight-chain or branched alkoxy or alkoxycarbonyl, each of which has up to 6 carbon atoms, or straight-chain or branched alkyl having up to 6 carbon atoms, or represents straight-chain or branched alkyl having up to 6 carbon atoms or trifluoromethyl, |

with the proviso that two of the substituents $R_1$-$R_4$ are always other than hydrogen, if appropriate in an isomeric form, and their salts.

2. Substituted cycloalkano[b]-indolesulphonamides of the formula (I) according to Claim 1 in which

| | |
|---|---|
| $R_1$, $R_2$, $R_3$ and $R_4$ | are identical or different and represent hydrogen, fluorine or chlorine, and |
| X | represents phenyl which is optionally up to tri-substituted by identical or different substituents from the series comprising fluorine, chlorine, bromine, cyano, trifluoromethyl, trifluoromethoxy or straight-chain or branched alkyl, alkoxy or alkoxycarbonyl, each of which has up to 4 carbon atoms, |

with the proviso that two of the substituents $R_1$ to $R_4$ are always other than hydrogen, if appropriate in an isomeric form, and their salts.

3. Substituted cycloalkano[b]-indolesulphonamides according to Claims 1 and 2 for controlling diseases.

4. Process for the preparation of substituted cycloalkano[b]-indolesulphonamides according to Claims 1 and 2, characterized in that

[A] compounds of the general formula

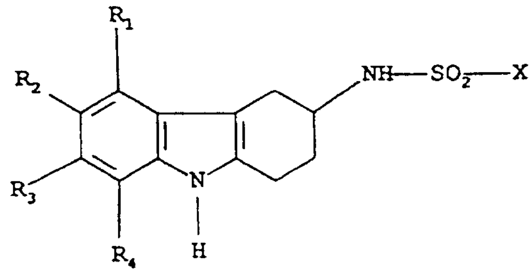

in which
$R_1$, $R_2$, $R_3$, $R_4$ and X have the abovementioned meaning, are initially reacted with acrylonitrile in inert solvents, if appropriate in the presence of a base, to give the corresponding cyanoethyl compounds and the product is then hydrolysed to give the corresponding acids,
or in that
[B] compounds of the general formula

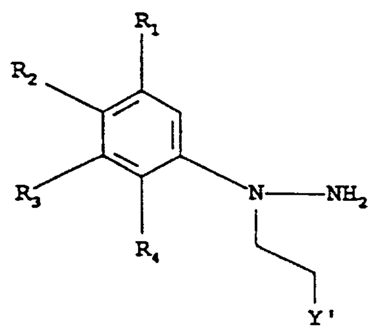

in which
$R_1$, $R_2$, $R_3$ and $R_4$ have the abovementioned meaning, and
Y' represents ($C_1$-$C_4$)-alkoxycarbonyl or cyano,
are reacted with cycloalkanosulphonamides of the general formula

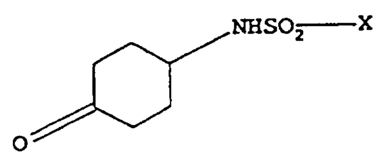

in which
X has the abovementioned meaning,
in inert solvents, if appropriate in the presence of a catalyst, and the esters are hydrolysed by a customary method, if desired, the isomers are separated, and, if salts are prepared, the product is reacted with a suitable base.

**5.** Medicaments containing at least one substituted cycloalkano[b]-indolesulphonamide according to Claims 1 and 2.

**6.** Medicaments according to Claim 5 for the treatment of thromboses, thromboembolisms, ischaemias, asthma and allergies, and for the prophylaxis of myocardial infarct.

**7.** Medicaments according to Claim 5 containing 0.5 to 90 % by weight of the substituted cycloalkano[b]-indolesulphonamides according to Claim 1.

8. Process for the preparation of a medicament according to Claim 6, characterized in that compounds according to Claim 1 are brought into a suitable form for administration, if appropriate with the aid of customary auxiliaries and excipients.

9. Use of the substituted cycloalkano[b]-indolesulphonamides according to Claims 1 and 2 for the preparation of medicaments.

10. Use of the substituted cycloalkano[b]-indolesulphonamides according to Claim 1 for the preparation of medicaments for the treatment of thromboses, thromboembolisms, ischaemias, asthma and allergies, and for the prophylaxis of myocardial infarct.

**Claims for the following Contracting State : ES**

1. Process for the preparation of substituted cycloalkano[b]-indolesulphonamides of the formula

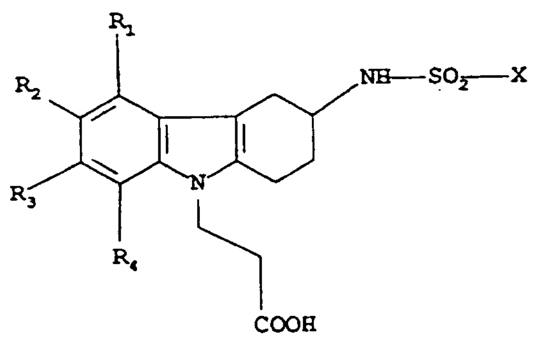

in which

$R_1$, $R_2$, $R_3$ and $R_4$ are identical or different and represent hydrogen, fluorine, chlorine, bromine or iodine, and

X represents phenyl which can optionally be up to tetrasubstituted by identical or different substituents from the series comprising nitro, fluorine, chlorine, bromine, iodine, cyano, trifluoromethyl, trifluoromethoxy, hydroxyl, carboxyl, phenyl, phenoxy, straight-chain or branched alkoxy or alkoxycarbonyl, each of which has up to 6 carbon atoms, or straight-chain or branched alkyl having up to 6 carbon atoms, or represents straight-chain or branched alkyl having up to 6 carbon atoms or trifluoromethyl,

with the proviso that two of the substituents $R_1$-$R_4$ are always other than hydrogen,
if appropriate in an isomeric form, and their salts,
characterized in that

[A] compounds of the general formula

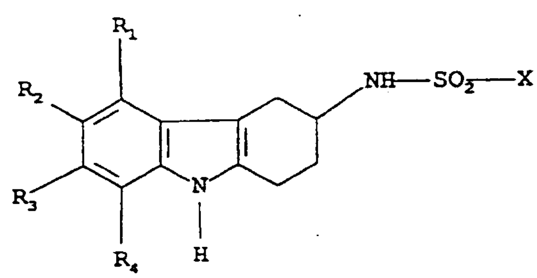

in which

$R_1$, $R_2$, $R_3$, $R_4$ and X have the abovementioned meaning, are initially reacted with acrylonitrile in inert solvents, if appropriate in the presence of a base, to give the corresponding cyanoethyl compounds and the product is then hydrolysed to give the corresponding acids,
or in that

[B] compounds of the general formula

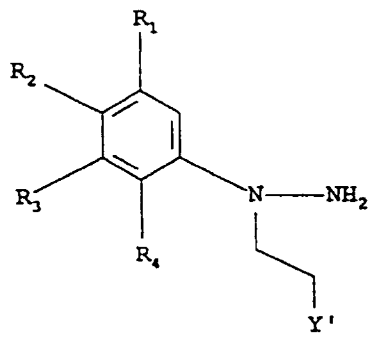

in which
$R_1$, $R_2$, $R_3$ and $R_4$ have the abovementioned meaning, and
Y' represents ($C_1$-$C_4$)-alkoxycarbonyl or cyano, are reacted with cycloalkanosulphonamides of the general formula

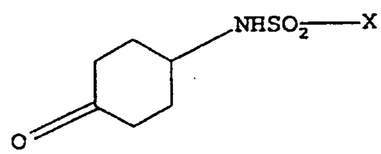

in which
X has the abovementioned meaning,
in inert solvents, if appropriate in the presence of a catalyst, and the esters are hydrolysed by a customary method, if desired, the isomers are separated, and, if salts are prepared, the product is reacted with a suitable base.

2. Process according to Claim 1, characterized in that the reaction with acrylonitrile is carried out in a temperature range from 0 - 150 °C.

3. Process according to Claim 1, characterized in that the esterification and amidation reactions are carried out via the active step of the acid halides.

4. Process according to Claim 1, characterized in that the processes are carried out under atmospheric pressure.

**Revendications**

**Revendications pour les Etats contractants suivants : AT, BE, CH, DE, DK, FR, GR, GB, IT, LU, LI, NL, SE**

**1.** Cycloalcano[b]-indolsulfonamides substitués de formule (I)

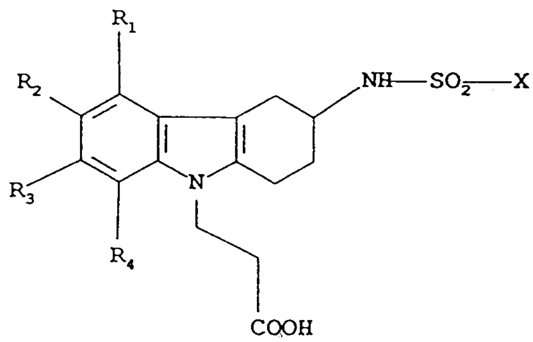

dans laquelle

$R_1$, $R_2$, $R_3$ et $R_4$ sont identiques ou différents et représentent l'hydrogène, le fluor, le chlore, le brome ou l'iode, et

X est un groupe phényle qui peut porter le cas échéant jusqu'à 4 substituants, identiques ou différents, nitro, fluoro, chloro, bromo, iodo, cyano, trifluorométhyle, trifluorméthoxy, hydroxy, carboxy, phényle, phénoxy, alkoxy ou alkoxycarbonyle à chaîne droite ou ramifiée ayant chacun jusqu'à 6 atomes de carbone, ou alkyle linéaire ou ramifié ayant jusqu'à 6 atomes de carbone, ou représente un groupe alkyle linéaire ou ramifié ayant jusqu'à 6 atomes de carbone ou un groupe trifluorométhyle,

sous réserve que parmi les substituants $R_1$ à $R_4$, il y en ait toujours deux d'entre eux différents de l'hydrogène,

le cas échéant sous une forme isomère, et leurs sels.

**2.** Cycloalcano[b]indolsulfonamides de formule suivant la revendication 1,
dans laquelle

$R_1$, $R_2$, $R_3$ et $R_4$ sont identiques ou différents et représentent l'hydrogène, le fluor ou le chlore, et

X est un groupe phényle qui porte le cas échéant jusqu'à 3 substituants, identiques ou différents, fluoro, chloro, bromo, cyano, trifluorométhyle, trifluorométhoxy ou alkyle, alkoxy ou alkoxycarbonyle linéaire ou ramifié avec chacun jusqu'à 4 atomes de carbone,

sous réserve que parmi les substituants $R_1$ à $R_4$, il y ait toujours deux d'entre eux différents de l'hydrogène,

le cas échéant sous une forme isomère, et leurs sels.

**3.** Cycloalcano[b]indolsulfonamides substitués suivant les revendications 1 et 2, destinés à combattre des maladies.

**4.** Procédé de production de cycloalcano[b]indolsulfonamides substitués suivant les revendications 1 et 2, caractérisé en ce que

[A] On fait tout d'abord réagir des composés de formule générale

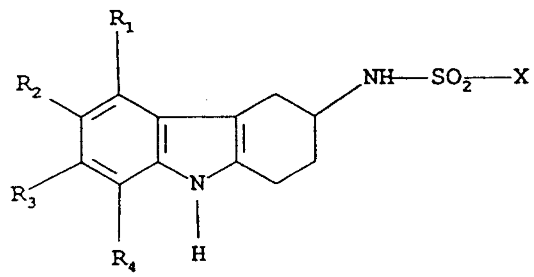

dans laquelle
$R_1$, $R_2$, $R_3$, $R_4$ et X ont la définition indiquée, avec l'acrylonitrile, le cas échéant en présence d'une base dans des solvants inertes pour former les composés cyanéthyliques correspondants que l'on saponifie ensuite en les acides correspondants,
ou bien
[B] On fait réagir des composés de formule générale

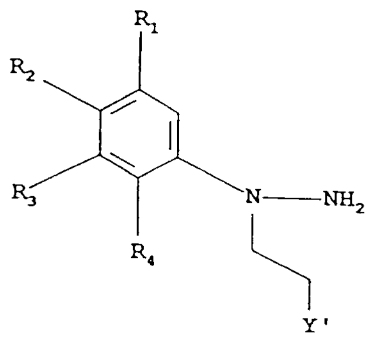

dans laquelle
$R_1$, $R_2$, $R_3$ et $R_4$ ont la définition indiquée, et
Y' est un groupe (alkoxy en $C_1$ à $C_4$)carbonyle ou cyano,
avec des cycloalcanosulfonamides de formule générale

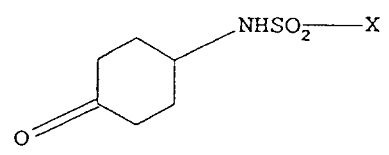

dans laquelle
X a la définition indiquée
dans des solvants inertes, le cas échéant en présence d'un catalyseur, et on saponifie les esters par un procédé classique, on sépare éventuellement les isomères et, dans le cas de la production des sels, on les fait réagir avec une base correspondante.

**5.** Médicament contenant au moins un cycloalcano[b]indolsulfonamide substitué suivant les revendications 1 et 2.

**6.** Médicament suivant la revendication 5, destiné au traitement de thromboses, de thrombo-embolies, d'ischémies, de l'asthme et d'allergies ainsi que pour la prophylaxie de l'infarctus du myocarde.

**7.** Médicament suivant la revendication 5, contenant 0,5 à 90 % en poids des cycloalcano[b]-indolsulfonamides substitués suivant la revendication 1.

8. Procédé d'obtention d'un médicament suivant la revendication 6, caractérisé en ce qu'on met sous une forme d'application appropriée des composés suivant la revendication 1, le cas échéant à l'aide de substances auxiliaires et de supports classiques.

9. Utilisation des cycloalcano[b]indolsulfonamides substitués suivant les revendications 1 et 2 pour l'obtention de médicaments.

10. Utilisation des cycloalcano[b]indolsulfonamides substitués suivant la revendication 1 pour l'obtention de médicaments destinés au traitement de thromboses, de thrombo-embolies, d'ischémies, de l'asthme et d'allergies ainsi que pour la prophylaxie de l'infarctus du myocarde.

**Revendications pour l'Etat contractant suivant : ES**

1. Procédé de production de cycloalcano[b]indolsulfonamides substitués de formule

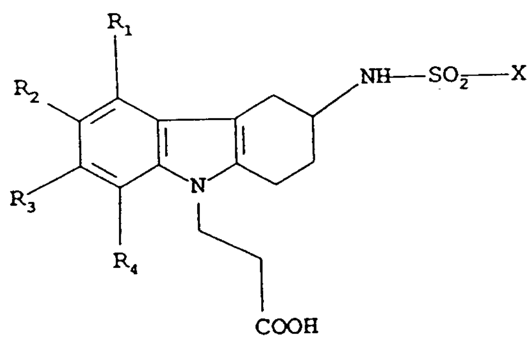

dans laquelle

| | |
|---|---|
| $R_1$, $R_2$, $R_3$ et $R_4$ | sont identiques ou différents et représentent l'hydrogène, le fluor, le chlore, le brome ou l'iode, et |
| X | est un groupe phényle qui peut porter le cas échéant jusqu'à 4 substituants, identiques ou différents, nitro, fluoro, chloro, bromo, iodo, cyano, trifluorométhyle, trifluorméthoxy, hydroxy, carboxy, phényle, phénoxy, alkoxy ou alkoxycarbonyle à chaîne droite ou ramifiée ayant chacun jusqu'à 6 atomes de carbone, ou alkyle linéaire ou ramifié ayant jusqu'à 6 atomes de carbone, ou représente un groupe alkyle linéaire ou ramifié ayant jusqu'à 6 atomes de carbone ou un groupe trifluorométhyle, |

sous réserve que parmi les substituants $R_1$ à $R_4$, il y en ait toujours deux d'entre eux différents de l'hydrogène,
le cas échéant sous une forme isomère, et leurs sels,
caractérisé en ce que

[A] On fait tout d'abord réagir des composés de formule générale

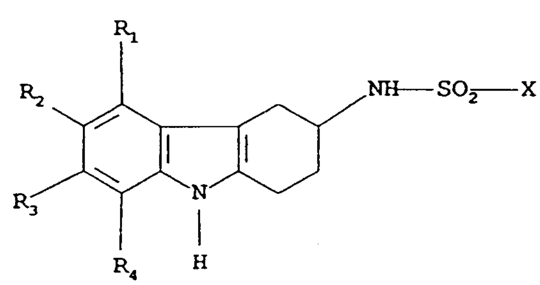

dans laquelle
$R_1$, $R_2$, $R_3$, $R_4$ et X ont la définition indiquée, avec l'acrylonitrile, le cas échéant en présence d'une base dans des solvants inertes pour former les composés cyanéthyliques correspondants que l'on saponifie ensuite en les acides correspondants,
ou bien

[B] On fait réagir des composés de formule générale

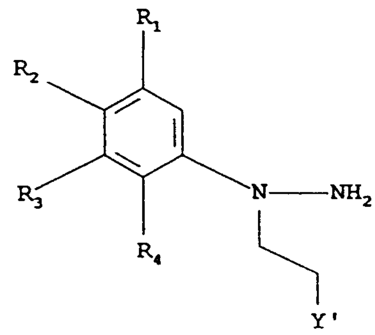

dans laquelle
$R_1$, $R_2$, $R_3$ et $R_4$ ont la définition indiquée, et
Y' est un groupe (alkoxy en $C_1$ à $C_4$)carbonyle ou cyano,
avec des cycloalcanosulfonamides de formule générale

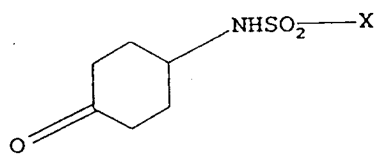

dans laquelle
X a la définition indiquée
dans des solvants inertes, le cas échéant en présence d'un catalyseur, et on saponifie les esters par un procédé classique, on sépare éventuellement les isomères et, dans le cas de la production des sels, on les fait réagir avec une base correspondante.

**2.** Procédé suivant la revendication 1, caractérisé en ce qu'on conduit la réaction avec l'acrylonitrile dans une plage de températures de 0 à 150 °C.

**3.** Procédé suivant la revendication 1, caractérisé en ce qu'on conduit l'estérification et l'amidation en passant par le stade actif des halogénures d'acides.

**4.** Procédé suivant la revendication 1, caractérisé en ce qu'on le met en oeuvre à la pression normale.